(19)

**Europäisches Patentamt**

**European Patent Office**

**Office européen des brevets**

(11) **EP 4 614 813 A1**

(12) **EUROPEAN PATENT APPLICATION**

(43) Date of publication:
**10.09.2025 Bulletin 2025/37**

(21) Application number: **24161850.3**

(22) Date of filing: **06.03.2024**

(51) International Patent Classification (IPC):
*H03K 3/57* ^(2006.01)     *A61N 2/02* ^(2006.01)

(52) Cooperative Patent Classification (CPC):
**H03K 3/57; A61N 2/006; A61N 2/02**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Universität der Bundeswehr München 85579 Neubiberg (DE)**

(72) Inventor: **Schwitzgebel, Florian 93047 Regensburg (DE)**

(74) Representative: **Lucke, Andreas Boehmert & Boehmert Anwaltspartnerschaft mbB Pettenkoferstrasse 22 80336 München (DE)**

(54) **A METHOD AND SYSTEM FOR GENERATING A PREDETERMINED ELECTROMAGNETIC PULSE**

(57)     Disclosed herein is a method of generating a predetermined electromagnetic pulse using a voltage source (32) comprising a chain of N switchable modules (12), the method comprising the steps of: determining a desired voltage pulse waveform which is suitable for generating said predetermined electromagnetic pulse when applied to said load (33); determining an individual start module voltage for each of said N modules, based on said desired voltage pulse waveform; charging an energy storage device (18) of each module (12) such as to acquire the corresponding determined start module voltage, and generating a time-dependent output voltage at the voltage source resembling said desired voltage pulse waveform by selectively switching said switchable modules (12).

Fig. 9

**Description**

FIELD OF THE INVENTION

The invention relates to method of and device for generating a predetermined electromagnetic pulse. More particularly, the invention relates to methods and devices for generating stimulation pulses for inductive neuron stimulation, in particular magnetic stimulation pulses for transcranial magnetic stimulation (TMS).

BACKGROUND AND STATE OF THE ART

Magnetic stimulation, in which certain cells are stimulated by externally acting electromagnetic fields in the body tissue, is regarded as the only currently painless non-invasive method for stimulating neurons in the brain of a patient. In addition, it has also increasingly been used in the periphery of the nervous system in medical rehabilitation.

Magnetic stimulation is usually based on the principle of magnetic induction. A conductive coil, the so-called treatment coil or stimulation coil, is placed in the vicinity of a patient and a current that varies over time flows through it, so that a correspondingly time-varied magnetic field is established, which penetrates the tissue of the patient and induces electrical fields that vary over time therein. These fields stimulate the neurons. An advantage of inductive magnetic stimulation is that it does not require physical contact with the coil, since the magnetic field of the coil also reaches body tissue at a certain distance from the coil. In addition, in contrast to electrical stimulation via electrodes, the method is virtually completely free of pain, since high current densities in areas with a high nociceptor density, for example the skin, are avoided. For these reasons, the method is also well suited for stimulating deep-lying tissue structures, for example the cerebral cortex, through the cranial bone, and for painless muscle stimulation.

The potential fluctuations on the neuron membrane required for stimulation lie in the range of only a few millivolts. Conventional stimulation devices, which usually operate according to the principle of resonant circuits, nevertheless require pulse powers in the megawatt range with a waste heat of sometimes several kilowatts. The stimulation coil heats up so much that the duration of use in a typical treatment session is often limited to only a few minutes. Furthermore, the power requirement greatly limits the practical usability of the magnetic stimulation, above all because of the required capacity of the power supply units and the high pulse rates. In prior art protocols of neuromodulation, up to four stimulators are coupled and operated alternately for a single patient. However, such stimulation devices are large, heavy and unwieldy and currently hardly suitable for operation away from large clinics.

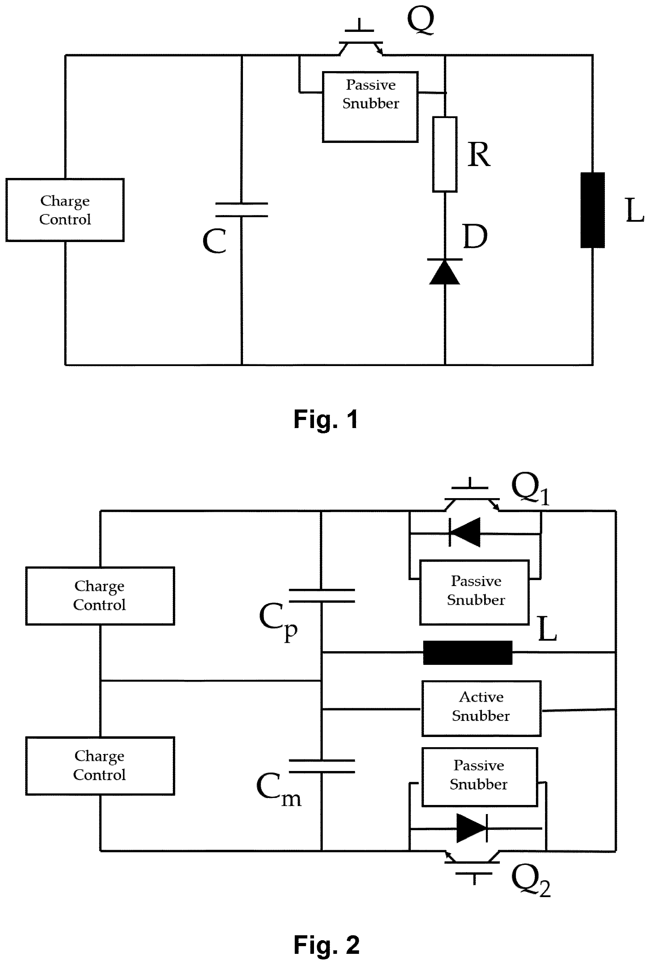

A conventional circuit topology for generating controlled, monophasic magnetic pulses of high intensity for transcranial magnetic stimulation is shown in FIG. 1. It comprises a resonant circuit composed of a high-voltage capacitor C and a stimulation coil L, which are connected via a switch Q, for example a transistor. A charging circuit charges the capacitor C to a voltage of several 1000 V. The energy content of the capacitor can be a few 100 J. Closing of the switch Q then initiates the current flow through the coil L and generates the stimulation field. However, most of the energy is lost as waste heat via the resistor R.

A further development which limits the waste heat losses is the circuit topology shown schematically in FIG. 2 taken from A. V. Peterchev et al., "Repetitive Transcranial Magnetic Stimulator with Controllable Pulse Parameters", Journal of Neural Engineering, Vol. 8, No. 3 (2011) 036016. In this half-bridge configuration, the stimulation coil L is connected alternately to the high-voltage capacitors Cp and Cm via switches $Q_1$ and $Q_2$. The energy drawn from the first capacitor Cp when closing the switch Qibut not converted into the magnetic pulse in the coil L can be fed back at least partially to the second capacitor Cm. When the switch $Q_2$ is subsequently closed, the capacitor Cm then conversely feeds the capacitor Cp, with the result that the waste heat losses are lower in comparison with the switching configuration in FIG. 1. In addition, the use of two independent capacitors Cp and Cm with respectively separate charging circuits increases the flexibility in the generation of the pulse shapes.

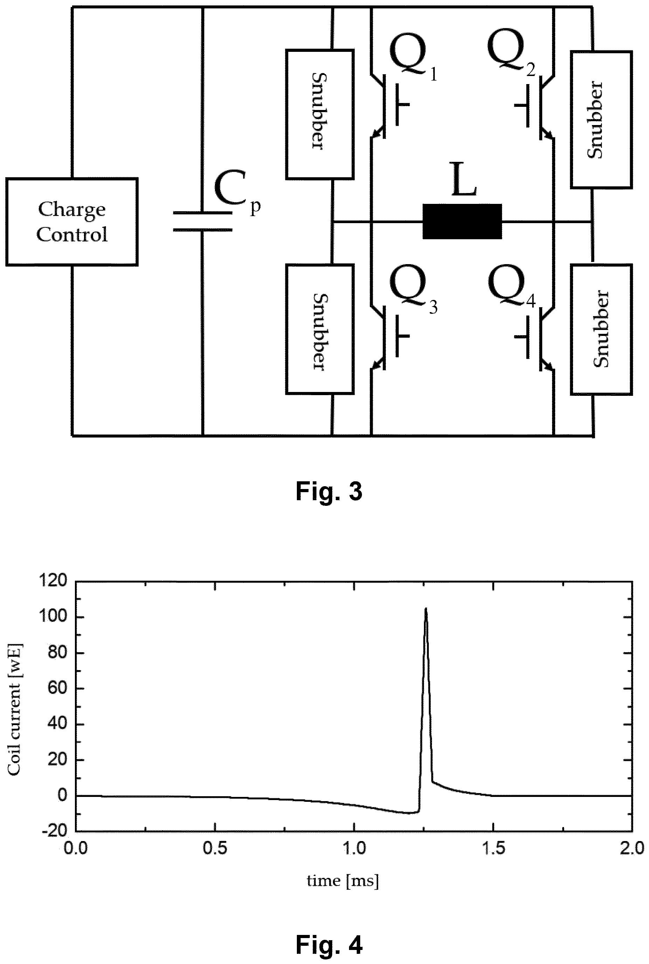

A further development of the half-bridge circuit in FIG. 2 to form a full bridge is shown in FIG. 3 and described in further details in A. V. Peterchev, "Circuit Topology Comparison and Design Analysis for Controllable Pulse Parameter Transcranial Magnetic Stimulators", Proceedings of the 5th International IEEE EMBS Conference on Neural Engineering 2011, p. 646. In contrast to the configuration in FIG. 2, the full-bridge circuit comprises four switches Q1, Q2, Q3, Q4, with the result that two different voltage levels can be applied to the stimulation coil L. If the charging voltage of the capacitor Cp is denoted by V, the stimulation coil L can therefore be switched between the voltages V, -V and 0. If this circuit configuration is supplemented by a second capacitor branch (with charging voltage V'), the differential voltage V-V' can also be set at the coil. Further details on the pulse shapes which can be achieved in this way can be found in the patent specifications U.S. Pat. No. 7,753,836 B2 and U.S. Pat. No. 7,946,973 B1.

The circuit configurations in FIGS. 2 and 3 increase the flexibility in the formation of magnetic pulses and at the same time reduce the heat losses. However, the generation of sufficiently high potential fluctuations on the neuron membrane still requires very high pulse powers with these circuits.

In the study S. M. Götz et al., "Analysis and Optimisation of Pulse Dynamics for Magnetic Stimulation", arxiv:

1106.3452v1, 17 June 2011, it was recognized by complex simulation calculations that the energy efficiency of the magnetic stimulation can be increased decisively by skillful selection of the pulse shape. In a numerical optimization for the magnetic stimulation of A$\alpha$ fibers and upper motor neurons, which belong to the pyramidal neurons and represent a preferred target of magnetic stimulation, a leading edge with relatively slow dynamics, which leads the main pulse and is opposite to it in the current direction, has proven to be particularly advantageous and energy-efficient. A corresponding time profile of the coil current is shown schematically in FIG. 4, which is taken from *Götz et al.* The leading edge 10, which introduces the actual main pulse 12 with a drop of the carrier into the negative, can be seen clearly in FIG. 4. As a result of the drop of the carrier into the negative, the leading pulse enables a subsequent relatively long rising edge of the main pulse, which in turn enables a high induced electric field strength. At the same time, the peak currents, which have a quadratic effect on the power loss, are limited. The maximum value of the derivative, which represents a measure of the dynamics, can be more than one hundred times lower in the preliminary phase than the gradient values reached in the main pulse. The simulation calculations in *S. M. Götz et al.* have shown that, as a result of suitably shaped pulses, in particular as a result of the use of leading edges, the energy losses in the generation of the magnetic pulses can be reduced by a multiple in comparison with the methods used hitherto. However, none of the above conventional magnetic stimulation devices is set up to generate pulses of the shape which have proven to be particularly advantageous in the study.

In view of these difficulties, in an earlier application of the applicant, DE 10 2012101921 A1, a device for generating stimulation pulses for inductive neuron stimulation was suggested. The device comprises at least one stimulation coil and at least one first half-bridge circuit, the output terminal of which is electrically connected to the stimulation coil. The first half-bridge circuit is a multilevel half-bridge with at least three different voltage levels. In this prior patent application of the present applicant, it was recognized that these types of circuit topologies, are very well suited for inductive magnetic stimulation in order to generate energy efficient pulse-waveforms while at the same time allowing for increased flexibility in generating a large variety of desired pulse waveforms.

An even better flexibility can be obtained using modular multilevel converters (MMC). A modular multilevel converter has first been described in DE 102 17 889 A1, and is further discussed e.g. in S. Allebrod, R. Hamerski, and R. Marquardt, "New transformerless, scalable modular multilevel converters for hvdc-transmission," in 2008 IEEE Power Electronics Specialists Conference, June 2008, pp. 174-179. Such MMC consists of one or more converter arms which are built up from a series connection of modules, each having a capacitor acting as an energy storage device, an input and an output terminal as well as switches allowing for selectively connecting the capacitors of adjacent modules in series, or bypassing or "deactivating" the capacitors in the series connection. By selecting a subset of module capacitors that are connected in series at any instance of time, a desired total voltage across the converter arm can be established.

In the original MMC, the capacitors in each module were typically identical, and were intended to provide on average the same voltages during operation. In a recent patent publication, DE10 2017 108 099 A1 of the same applicant, a new type of modular multilevel converter was disclosed, which may be referred to as "exponential modular multilevel converter" (EMMC). The EMMC allows for reducing cost and complexity as compared to the original MMC. The reduction in costs is mainly because a much smaller number of modules is needed in the EMMC to provide a same output voltage divided in a same number of output voltage stages. For further details of the EMMC and an explanation of its operating principles, reference is made to DE10 2017 108 099 A1, the full content of which is incorporated herein by reference, as well as to Kuder, M., Kersten, A., Bergmann, L. et al (2019). Exponential Modular Multilevel Converter for Low Voltage Applications. 2019 21st European Conference on Power Electronics and Applications, EPE 2019 ECCE Europe: 1-11. http://dx.doi.org/10.23919/EPE.2019.8915156

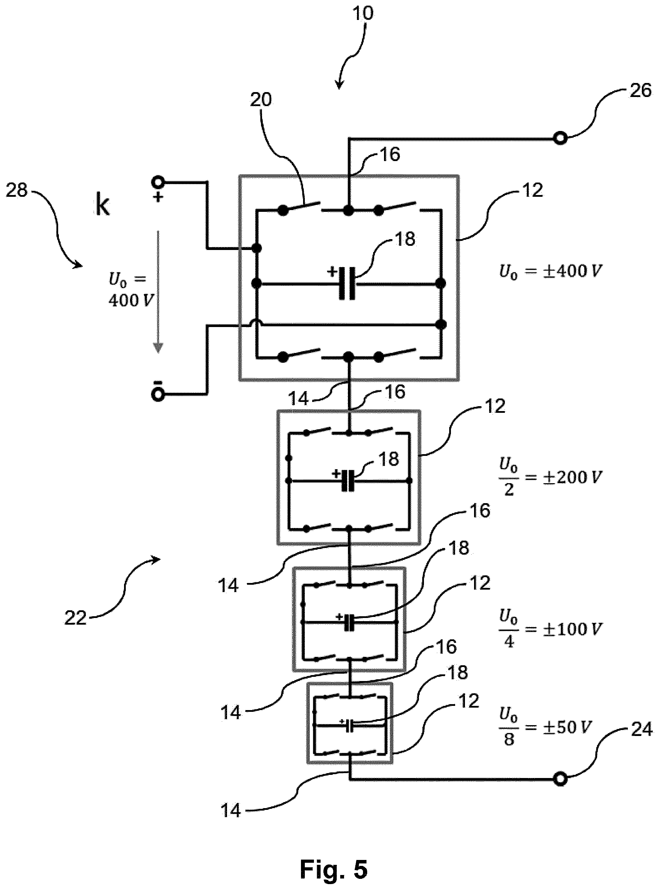

An example of an EMMC 10 is shown in Fig. 5. The EMMC 10 of Fig. 5 comprises four cascaded modules 12 each having a first terminal, a second terminal, a capacitor 18 and a plurality of switches, which in the embodiment shown establish a full H-bridge topology. The cascaded connection of the four modules 12 forms a converter arm 22 having a first end 24 and a second end 26.

For each two adjacent modules 12, the first terminal of one module 12 is connected with the second terminal of the other module 12. By operating the switches, it is possible to connect the capacitor 18 of each module 12 selectively in series and in anti-series with the capacitor 18 of the adjacent module. The current voltages of the capacitors 18 connected in series and anti-series add up to a total voltage applied between the first and second ends 24, 26 of the converter arm 22. Moreover, the switches also allow the module 12 to acquire a "bypass" or "deactivated" state, in which the capacitor 18 is bypassed, or in other words, not part of the series/anti-series connection and not contributing to the total voltage between the first and second ends 24, 26 of the converter arm 22.

In the EMMC of Figure 5, the capacitor 18 of the uppermost module 12, also referred to as the "main module" in the following, is constantly connected to a DC voltage $U_o$ provided by a DC voltage source. The capacitors 18 of the other three modules are in operation kept on voltages decreasing at a power of two, i.e. $U_o/2^n$, with n = 1, 2, 3, leading to voltage levels of 200 V, 100 V and 50 V. The exponentially decreasing voltages of the additional modules 12 give rise to the name "exponential" MMC (EMMC).

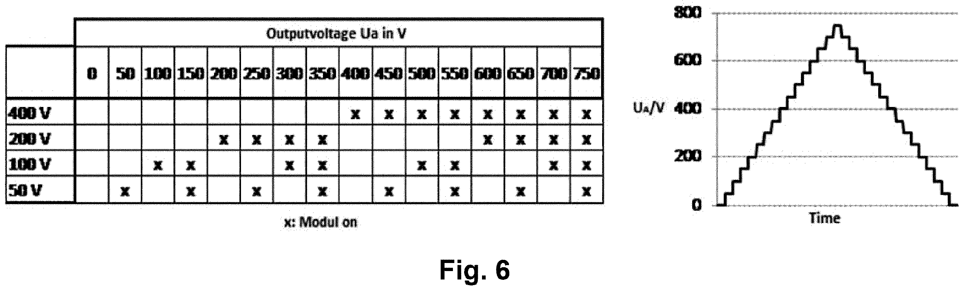

With the configuration shown, it is possible to map an output voltage curve of the converter arm 22 with a step height of 50 V. The minimum step height corresponds to the voltage of the "smallest" additional module, i.e. the module 12 having the capacitor 18 with the smallest nominal voltage. This is immediately understandable from Fig. 6, in which only series connections (i.e. no anti-series connections) of selected modules 12 are illustrated. The table on the left shows for each output voltage between 0 V and 750 V which of the modules 12 are "on", i.e. incorporated in the series connections, which is marked by the symbol "x", with the remaining modules 12 being in the bypass state. The diagram on the right of Fig. 6 shows an exemplary waveform of voltages between the first and second ends 24, 26 of the converter arm 22 that can be obtained by successively switching the modules 12 to the states summarized in the table.

It is seen that in principle, voltages up to twice the main module voltage minus the smallest additional module voltage, in the shown case = 750 V, can be mapped for the positive case. To output negative voltages, the modules shown in Fig. 5 are operated inverted, i.e. with $-U_o, -U_o/2$ etc. By varying the step width, the system has a high degree of flexibility in approaching different output wave forms. An important application is the generation of stepped approximations to sine functions with essentially arbitrary frequencies, which can be used to e.g. power electric motors. However, one of the great advantages of MMCs and EMMCs in particular is that they allow for generating practically arbitrary time-dependent output voltages, including desired voltage pulse waveforms which are suitable for generating a predetermined magnetic pulse when applied to a coil device for the purposes of medical magnetic stimulation. This understanding was exploited in a yet further patent application by the present applicant, DE 10 2017 108 084 A1, where an EMMC is used as a voltage source for generating magnetic stimulation pulses, and in particular for TMS purposes.

In the operation described so far, all of the active modules 12 are connected with same polarity, i.e. all positive or all negative. This can typically only be carried out for short periods of time, since a module that contributes with its voltage to the total voltage will be discharged by the load current, while in the art, the operation of MMCs always relies on fixed, predetermined or "set" module voltages to allow for establishing the multiple voltage levels. In other words, keeping the module voltages (i.e. the voltage of the energy storage device within the module) at their predetermined or "set" voltage levels is a central aspect of state-of-the-art MMC and EMMC operation. For this purpose, in MMC and EMMC operation, the module energy storage devices will be alternatingly charged and discharged in operation. Namely, an individual module that is connected with opposite polarity to the total voltage over the converter arm 22 will be charged. Accordingly, by switching selected module capacitors 18 in anti-series with the predominant part of the modules (as far as the voltage contribution is concerned), this anti-serially connected module capacitor 18 can be charged upon operation, and since its voltage is in this case subtracted from the total voltage, this operation also allows for a larger variety of establishing desired output voltages.

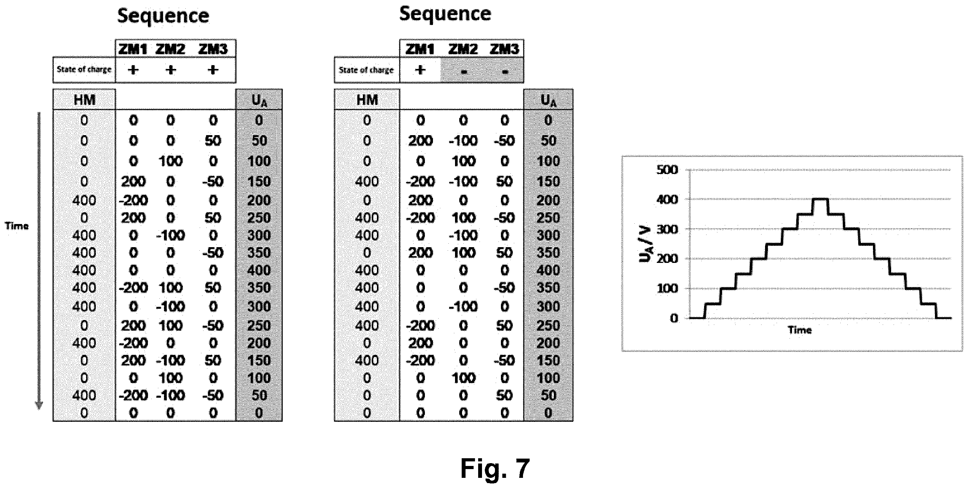

This is illustrated in Fig. 7, where on the right a waveform oscillating between 0 V and 400 V is shown, and in the table on the left, possible output voltages of the individual modules 12 for establishing a total voltage "$U_A$" are summarized, wherein the main module is designated by "HM", and the additional modules are designated by "ZM1", "ZM2", and "ZM3", respectively. As seen from the tables, each total voltage level $U_A$ other than 0 V and 400 V can be generated by two or more different switching states.

More precisely, both tables in Fig. 7 illustrate the positive rise and fall of the voltage with time as shown in the diagram on the right. The first sequence summarized in the left table starts with sufficiently charged capacitors (state of charge "+"), while in the second sequence (right table) the capacitors of the additional modules ZM2 (100 V) and ZM3 (50 V) are not sufficiently charged at the beginning (state of charge "-") and should therefore be switched anti-serially and thus inverted into the path at the first use. In the chronological sequence of the individual switching states, the alternating use of the additional modules in charging and discharging mode can be seen. The main module HM can be positively switched on at any time if required, since it is connected with the DC current source as seen in Fig. 5. The logic for determining the next switching state follows the principle of deliberately building up a voltage deficit and increasing it exponentially until the main module compensates at the latest.

In contrast to the "all positive case" shown in Fig. 6, however, it is no longer always possible to exceed the basic module voltage of 400 V as the maximum voltage. Instead, in the combined charging/discharging variant of Fig. 7, where at least some of the capacitors 18 are connected in anti-series and in which selected capacitors 18 are hence connected with opposite polarity than the total voltage $U_A$, the maximum total voltage is limited to the main module voltage, in this case 400 V.

In view of the possibility to invert selected capacitors 12 for charging, the requirements for the capacities of the capacitors 12 are greatly diminished. The reason is that different module states can be switched one after the other while still leading to the same total voltage $U_A$, thereby allowing the charge or voltage states of all module capacitors to be balanced at any time.

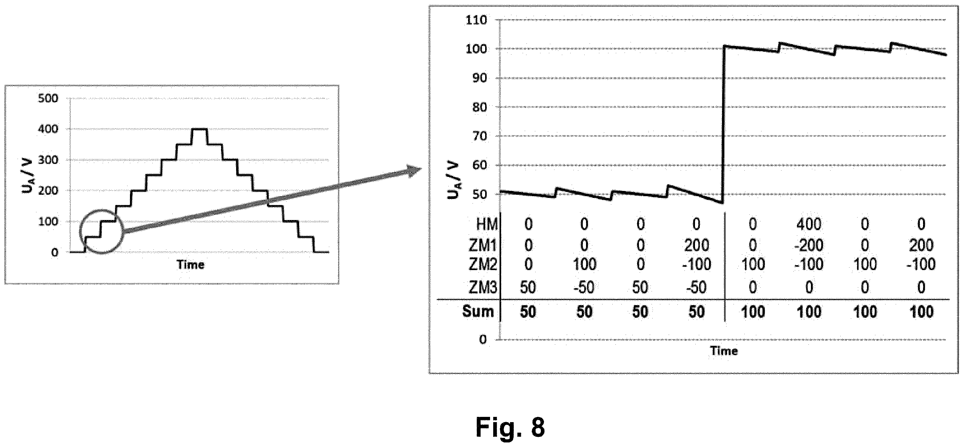

Fig. 8 shows a detailed example of the first two voltage stages of the waveform of Fig. 7, namely 50 V and 100 V, each with four different switching states. Within the individual switching states, both the discharging of positively switched and the charging of negatively switched capacitors result in a continuous voltage drop, with the voltage tolerance of the individual capacitors being set to $\pm$ 1V, for example. The possible number of intermediate stages is only limited by an upper limit of the reasonably possible switching frequency.

As is explained in the aforementioned DE 10 2017 108 084 A1, the EMMC can in many respects be regarded as ideal for use as a voltage source for generating stimulation pulses, in particular magnetic stimulation pulses for TMS applications. One of the advantages is that that the energy storage device voltages can be quite easily kept at their set values, using the operation as indicated above, without requiring large capacitors (i.e. having large capacitance) as voltage storage devices. Indeed, a closer inspection reveals that even with less ambitious voltage management for the individual module voltages than the one described above, in many applications, where the EMMC is connected to an inductive load (as is the case in a stimulation coil), the EMMC has a "self-stabilizing" effect with respect to the module voltages. This is because in the switching control of the EMMC, the modules will always be treated as having the set voltage, with the result that a module that happens to currently exceed this set voltage will quasi automatically be discharged to a higher degree in operation, and a module that has a voltage deficit (but is treated in the control as having the set voltage) will be charged by the inductive load in operation. In other words, as long as there is frequent switching of the modules, the module voltages will practically automatically stabilize.

A further advantage of the EMMC - as compared to a classical MMC with uniform module voltage levels - is that it allows for a much larger number of output voltage levels for a given number of modules. Namely, while the classical MMC with a number of N modules of uniform voltage allows for $2 \cdot N + 1$ output voltage levels (N positive, N negative and zero), an EMMC with N modules allows for $2^N + 1$ output levels. In this count of output voltage levels, the highest voltage is not the sum of all module voltages, but the voltage of the module having the highest module voltage, i.e. the module that was referred to as the "main module" above. This is because with this upper boundary, the recharging of the module energy storage devices during operation can be ensured. Even though in this count a number of in principle accessible voltage levels of the EMMC are disregarded, it is still seen that for a given number of modules, the number of output voltage levels is higher (and with increasing number N of modules dramatically higher) than in case of the classical MMC. This means that the step height between consecutive output voltage levels of the EMMC can be much lower than in a classical MMC, which in turn allows to more closely resemble the desired voltage pulse.

While the increased number of output voltage levels provided by an EMMC is rightly highlighted in DE 10 2017108 084 A1 as one of the particular advantages for its use in generating stimulation pulses, the EMMC is nevertheless still bound to a limited set of discrete output voltages, which hence prevents continuous or "smooth" output voltage waveforms. This issue is addressed in the aforementioned DE 10 2017108 084 A1, and it is suggested to either provide for an additional filter circuit for smoothing the stepped voltage levels, or to add an analogue module to the series connection of modules which allows for providing continuous voltages that can be used to interpolate between the steps.

SUMMARY OF THE INVENTION

A problem underlying invention is to provide a method and apparatus for generating a predetermined electromagnetic pulse using a voltage source and a load connected thereto, which allows for precisely resembling a desired voltage pulse waveform with simple means.

This problem is solved by a method according to claim 1 and a system according to claim 13. Preferable embodiments are defined in the dependent claims.

According to a first aspect of the invention, a method of generating a predetermined electromagnetic pulse using a voltage source and a load connected thereto is provided. Herein, the predetermined electromagnetic pulse may be a magnetic stimulation pulse and the load may be a stimulation coil device.

The voltage source comprises a chain of modules, in which a number of N modules are connected in series, with $N \geq 3$, and wherein a voltage across said chain of modules represents an output voltage of the voltage source to be applied to said load. Each of said modules comprises an energy storage device having a module voltage and a plurality of switching devices. The switching devices are configured to allow for selectively establishing each of the following at least three operating states:

- a series connection state, in which the energy storage device is connected to introduce its module voltage to the voltage path along the chain of modules with normal polarity to thereby positively contribute to the output voltage of the voltage source,

- an anti-series connection state, in which the energy storage device is connected to introduce its module voltage to the voltage path along the chain of modules with reversed polarity to thereby negatively contribute to the output voltage of the voltage source, and

- a bypass state, in which the module provides a conductive path therethrough without the energy storage device contributing to the output voltage of the voltage source.

Note that the terms "normal" and "reverse" polarity are merely used to distinguish the two polarization directions in which the energy storage device of the module can be connected in the chain of modules.

The method comprises the steps of:

- determining a desired voltage pulse waveform which is suitable for generating said predetermined electromagnetic pulse when applied to said load,

- determining an individual start module voltage for each of said N modules, based on said desired voltage pulse waveform,

- charging the energy storage devices of each module such as to acquire the corresponding start module voltage,

- generating a time-dependent output voltage at the voltage source resembling said desired voltage pulse waveform by selectively switching the switching devices of each of the modules to acquire a selected one of said at least three operating states as a function of time, and applying said time-dependent output voltage to said load.

Herein the feature "switching ... as a function of time" means that for each of the modules, the operating state may and typically will change over time, to thereby generate the likewise time-dependent output voltage. As mentioned before, the electromagnetic pulse can for example be a magnetic pulse which is generated by applying the voltage from the voltage source to the coil device. Knowing the impedance of the coil device ("the load"), it is hence possible to determine a voltage pulse waveform which, when applied to the coil device, generates suitable currents for generating the predetermined magnetic pulse. This voltage pulse waveform therefore is referred to as the "desired voltage pulse waveform", because the aim is to generate this voltage pulse waveform as accurately as possible using the voltage source.

Finally, said step of determining the individual start module voltage for each of said N modules comprises an optimization procedure, in which start module voltages are determined such as to reduce a deviation between the time-dependent output voltage and the desired voltage pulse waveform.

Accordingly, unlike the EMMC used as the voltage source in DE 10 2017 108 084 A1, where the module voltages always have a fixed value which is also maintained during the course of operation, in the present invention, the start module voltages are individually determined depending on the desired voltage pulse waveform, and are "optimized" for reducing a deviation between the time-dependent output voltage obtained when using these start voltages and the desired voltage pulse waveform.

Note that the term "optimization" indicates that the underlying goal is to determine individual start module voltages which lead to the smallest deviation between the time-dependent output voltage generated by the voltage source and the desired voltage pulse waveform, referred to as the "deviation" for short, but it does not mean that the set of start module voltages actually determined must be an "optimum" choice. Indeed, there is no straightforward way to determine the start module voltages that with certainty would lead to the smallest possible deviation. Instead, an "optimization procedure" as understood herein may be any procedure that for a given desired voltage pulse waveform leads to smaller deviations than those that are obtainable with an EMMC voltage source having the same number of modules. In other words, it is sufficient that the optimization procedure is such that with the thus determined start module voltages, one or both of the maximum and average deviation between the time-dependent output voltage and the desired voltage pulse waveform is less than the maximum and average deviation, respectively, obtainable with a voltage source having $2^N + 1$ evenly distributed voltage states between $-V_{pulse,max}$ and $+V_{pulse,max}$, preferably less than 75%, more preferably less than 50% of said maximum or average deviation, respectively, wherein $V_{pulse,max}$ is the maximum of the absolute value of the desired voltage pulse waveform..

Herein, the "maximum deviation" is the maximum of the absolute value of voltage difference between the time-dependent output voltage and the desired voltage waveform that occurs during the entire duration of the voltage pulse. The "average deviation" is the arithmetic mean of the absolute values of the voltage differences between the time-dependent output voltage and the desired voltage waveform determined at a number of discrete time steps, which may correspond to the switching frequency of the switching devices.

Note that the term "module" has a broad meaning and is mainly used to denote portions of the voltage source that allow for establishing the function as defined above, based on the aforementioned at least three operating states. These modules may in some embodiments be separate components that can be manufactured separately and can be connected in series such as to form the voltage source. However, the modules can also be formed by "virtual" portions or sections of a larger circuit that provide the structure and function as defined above.

In a preferred embodiment, during said step of generating said time-dependent output voltage, the energy storage devices are permitted to change their voltage due to energy transferred to the load and between modules, and this change of voltage is accounted for in the step of determining said set of individual start module voltages.

It is seen that although the hardware employed in this method is the same as that of an MMC/EMMC, the operation is fundamentally different. In an MMC, the starting point is a set of predetermined module voltages, typically evenly distributed in case of classical MMC and exponentially distributed in case of EMMC, and these module voltages are kept constant in operation by balancing operations, or by the above-mentioned inherent voltage stabilization of the EMMC. In operation of the classical MMC/EMMC, these predetermined and generally fixed module voltages are then combined, using a selected one of the three above-mentioned operation states (series connection state, anti-series connection state and bypass state) such as to produce an output voltage across the chain of modules that is the closest to a desired output voltage.

In contrast to this, according to the present invention, suitable start module voltages are determined individually for each desired voltage pulse waveform that is to be resembled by the voltage source. These individual start module voltages will hence not follow any neat pattern, and they will generally be different for each desired voltage pulse waveform to be resembled. Importantly, the start module voltages are chosen such that deviations (such as the aforementioned maximum deviation or average deviation) occurring during the *entire* course of the thus generated time-dependent output voltage (i.e. during the entire "voltage pulse") is reduced, even accounting for the changes of the individual module voltages during the course of the voltage pulse. This is conceptually very different from the ordinary MMC/EMMC operation.

In a preferred embodiment, said optimization procedure comprises the steps of

a) simulating the time-dependent output voltage that would be obtained with a given set of start module voltages,

b) determining a deviation indicator indicating a deviation of the simulated time-dependent output voltage from the desired voltage pulse waveform, and

c) modifying some or all of the start module voltages based on the determined deviation indicator.

Herein, the "deviation indicator" may e.g. be the aforementioned "maximum deviation". The "deviation indicator" should be an indicator that provides guidance for the modification of the start module voltages in step c) such that with the modified start module voltages, a smaller deviation can be expected to be obtained. The "maximum deviation" is a good choice for such indicator, because it does provide guidance to modify all or part of the start module voltages such that at least the maximum deviation can be reduced. However, other deviation indicators may be employed, as long as they provide suitable guidance towards improved start module voltages.

In the simulation in step a), the desired voltage pulse waveform may be discretized to a finite number of time steps, and for each time step, suitable operating states (i.e. series connection, anti-series connection, bypass) are determined for each module such as to generate an overall output voltage that is as close as possible to the desired voltage pulse waveform at that time step. Clearly, at each time step, the available module voltages will depend on the chosen set of start module voltages, but also on a possible change in the module voltages during the course of operation up to this time step. This is because unlike the classical MMC and EMMC, the module voltages are permitted to change under operation due to energy transferred to the load and between modules.

In a preferred embodiment, the simulation in said step a) comprises estimating the time-dependent current flow into the load and accounting for the change in module voltages therewith.

In a preferred embodiment, said steps a) to c) are conducted iteratively until a stop criterion is met. Such stop criterion may be a convergence criterion, and in particular, a criterion in which the average deviation of the simulated time-dependent output voltage from said desired voltage pulse waveform exceeds a threshold value or stops decreasing for a given number of successive iterations.

For the iterative optimization procedure of the start voltages to converge, it is important that in step c), some or all of the start module voltages are modified such that when starting out from the modified start voltages, a time-dependent output voltage of the voltage source can be generated that over the whole course thereof deviates less from the desired voltage pulse waveform. According to one embodiment, in step b), an error voltage $V_E$ is determined, which corresponds to the maximum deviation of the simulated time-dependent output voltage from said desired waveform, and in step c), the start module voltages are modified by subtracting different voltage values from all or at least some of the start module voltages which at least approximately add up to said error voltage $V_E$. Note that the "error voltage $V_E$" corresponds to the aforementioned "maximum deviation", and hence to the maximum of the absolute values of the voltage differences between the time-dependent output voltage and the desired voltage pulse waveform that occurs during the entire duration of the voltage pulse. $V_E$ is hence always a positive value, irrespective of whether the deviation is positive or negative.

Assuming that the output voltage of the voltage source exceeds the desired voltage at the time step where the maximum deviation occurs (output voltage "too high"), it is plausible that with the thus modified start voltages, this deviation can be significantly reduced. However, maybe less obviously, the thus modified start voltages are also favorable if the output voltage should be "too low". The reason is that in this case, the modules with the modified start voltages may be suitably connected in anti-series. For example, if there are 4 voltage modules having currently 50 V, and the desired voltage is 90 V, the closest possible value achievable is 100 V and hence "too high". This can be corrected by reducing the voltage of e.g. the first module by 10 V to 40 V, such that the desired voltage of 90 V can be achieved by connecting the first and second modules in series and bypassing the third fourth modules. However, if the desired voltage should happen to be 110 V, 100 V would still be the closest possible output value, but would be "too low". In this case too, the voltage of the first module could be reduced to 40 V, and could be connected in anti-series, while the second to fourth modules are connected in series, thereby leading to the desired 110 V.

Importantly, according to this embodiment, different voltage values shall be subtracted from all or at least some of the start module voltages, to thereby shift the relative voltages of the modules. Still, the subtracted voltage values should at least approximately add up to the error voltage $V_E$. Again, there are various different ways to distribute the subtracted parts of the error voltage $V_E$ that all lead to convergence. In one embodiment, in step c), the start module voltage of the n-th module is reduced by $V_E./(2 \cdot n)$, for n = 1 ... N. This modification of the start voltages has been found to lead to quick and reliable convergence of the time-dependent output voltage towards the desired voltage pulse waveform.

One particular advantage of the invention is that the generated time-dependent output voltage can resemble the desired voltage pulse waveform with high precision (i.e. very small average and maximum deviation) even for a comparatively small number N of modules. In preferred embodiments, $10 \geq N \geq 3$, preferably $8 \geq N \geq 4$.

In a preferred embodiment, said switching devices comprise transistor switching devices, in particular MOSFET or IGBT switching devices.

A further aspect of the invention relates to a system for generating a predetermined electromagnetic pulse, the system comprising a voltage source and a load connected thereto. The voltage source comprises a control device, and a chain of modules, in which a number of N modules are connected in series, with $N \geq 3$, and wherein a voltage across said chain of modules represents an output voltage of the voltage source to be applied to said load. Each of said modules comprises an energy storage device having a module voltage and a plurality of switching devices, wherein said switching devices are configured to allow for selectively establishing, under control of said control device, each of the following at least three operating states:

- a series connection state, in which the energy storage device is connected to introduce its module voltage to the voltage path along the chain of modules with normal polarity to thereby positively contribute to the output voltage of the voltage source,

- an anti-series connection state, in which the energy storage device is connected to introduce its module voltage to the voltage path along the chain of modules with reversed polarity to thereby negatively contribute to the output voltage of the voltage source, and

- a bypass state, in which the module provides a conductive path therethrough without the energy storage device contributing to the output voltage of the voltage source.

Said control device is further configured for receiving information representing a desired voltage pulse waveform which is suitable for generating said predetermined electromagnetic pulse when applied to said load, or for determining such desired voltage pulse waveform, wherein said control device is further configured for

- determining an individual start module voltage for each of said N modules, based on said desired voltage pulse waveform,

- controlling a charging of the energy storage device of each module such as to acquire the corresponding determined start module voltage, and

- controlling the switching elements such as to selectively switch the switching devices of each of the modules to acquire a selected one of said at least three operating states as a function of time, to thereby generate a time-dependent output voltage at the voltage source resembling said desired voltage pulse waveform.

Herein, said control device is configured for determining said individual start module voltage for each of said N modules using an optimization procedure, in which said start module voltages are determined such as to reduce a deviation between the generated time-dependent output voltage and the desired voltage pulse waveform.

In a preferred embodiment of the system, said predetermined electromagnetic pulse is a magnetic stimulation pulse and said load is a stimulation coil device.

In a preferred embodiment of the system, the optimization procedure is such that with the thus determined start module voltages, one or both of the maximum and average deviation between the time-dependent output voltage and the desired voltage pulse waveform is less than the maximum and average deviation, respectively, obtainable with a voltage source having $2^N + 1$ evenly distributed voltage states between $-V_{pulse,max}$ and $+V_{pulse,max}$, preferably less than 75%, more preferably less than 50% of said maximum or average deviation, respectively, wherein $V_{pulse,max}$ is the maximum of the absolute value of the desired voltage pulse waveform.

In a preferred embodiment of the system, said control device is configured to control said switches such that during said step of generating said time-dependent output voltage, the energy storage devices are permitted to change their voltage due to energy transferred to the load and between modules, wherein said control device is configured to account for this change of voltage in the step of determining said set of individual start module voltages.

In a preferred embodiment, said optimization procedure comprises the steps of

a) simulating the time-dependent output voltage that would be obtained with a given set of start module voltages,

b) determining a deviation indicator indicating a deviation of the simulated time-dependent output voltage from the desired voltage pulse waveform, and

c) modifying some or all of the start module voltages based on said determined deviation indicator.

In a related embodiment, the simulation in said step a), comprises estimating the time-dependent current flow into the load and accounting for the change in module voltages associated therewith.

In a further related embodiment, the control device is configured to conduct said steps a) to c) iteratively until a stop criterion is met, wherein preferably, said stop criterion is a convergence criterion, and in particular, a criterion in which the average deviation of the simulated time-dependent output voltage from said desired voltage pulse waveform exceeds a threshold value or stops decreasing for a given number of successive iterations.

In a preferred embodiment, the control device is configured to determine, in step b), an error voltage $V_E$, which corresponds to the maximum deviation of the simulated time-dependent output voltage from said desired voltage pulse waveform, said maximum deviation corresponding to the maximum of the absolute values of the voltage differences between the time-dependent output voltage and the desired voltage waveform that occurs during the entire duration of the voltage pulse, and to modify, in step c), the start module voltages by subtracting different voltage values from all or at least some of the start module voltages which at least approximately add up to said error voltage $V_E$.

In a related embodiment, said control device is configured to reduce, in step c), the start module voltage of the n-th module by $V_E/(2 \cdot n)$, for n = 1 ... N.

In a preferred embodiment of the system, $10 \geq N \geq 3$, preferably $8 \geq N \geq 4$.

In a preferred embodiment of the system, said energy storage device is a capacitor.

In a preferred embodiment of the system, said switching devices comprise transistor switching devices, in particular MOSFET or IGBT switching devices.

SHORT DESCRIPTION OF THE FIGURES

Fig. 1    shows a conventional resonant circuit for generating magnetic stimulation pulses.

Fig. 2    shows a conventional half bridge circuit with two different levels for generating magnetic stimulation pulses.

Fig. 3    is a full bridge circuit which in prior art has been used for generating TMS pulses.

Fig. 4    schematically shows the waveform of a coil current for particularly energy efficient magnetic stimulation, comprising a pre-pulse and a main pulse.

Fig. 5    is a schematic view of an exponential modular multilevel converter (EMMC).

Fig. 6    shows a waveform of an output voltage of the EMMC of Fig. 5 and a table of corresponding module switching states.

Fig. 7    shows a waveform of an output voltage of the EMMC of Fig. 5 and two tables of associated module switching states.

Fig. 8    shows a detailed example of the first two voltage stages of the waveform of Fig. 7, incorporating four different module switching states.

Fig. 9    is a schematic illustration of a system according to an embodiment of the invention.

Fig. 10    is a schematic illustration of a system according to another embodiment of the invention.

Fig. 11     shows a damped cosine as an exemplary desired voltage pulse waveform and a time-dependent output voltage resembling the same, generated with the method of the invention and using a voltage source having four modules.

Fig. 12     shows the same desired voltage pulse waveform as in Fig. 11 and a time-dependent output voltage resembling the same, generated with a conventional EMMC having four modules.

Fig. 13     shows the same desired voltage pulse waveform as in Fig. 11 and a time-dependent output voltage resembling the same, generated with the method of the invention and using a voltage source having three modules.

Fig. 14     shows the same desired voltage pulse waveform as in Fig. 11 and a time-dependent output voltage resembling the same, generated with a conventional EMMC having three modules.

Fig. 15     shows an un-damped cosine as an exemplary desired voltage pulse waveform and a time-dependent output voltage resembling the same, generated with the method of the invention and using a voltage source having four modules.

Fig. 16     shows the same desired voltage pulse waveform as in Fig. 15 and a time-dependent output voltage resembling the same, generated with a conventional EMMC having four modules.

Fig. 17     shows the same desired voltage pulse waveform as in Fig. 15 and a time-dependent output voltage resembling the same, generated with the method of the invention and using a voltage source having three modules.

Fig. 18     shows the same desired voltage pulse waveform as in Fig. 15 and a time-dependent output voltage resembling the same, generated with a conventional EMMC having three modules.

## DESCRIPTION OF THE PREFERRED EMBODIMENT

It is to be understood that both the foregoing general description and the following description are exemplary and explanatory only and are not restrictive of the methods and systems described herein. In this application, the use of the singular may include the plural unless specifically stated otherwise. Also, the use of "or" means "and/or" where applicable or unless stated otherwise. Those of ordinary skill in the art will realize that the following description is illustrative only and is not intended to be in any way limiting. Other embodiments will readily suggest themselves to such skilled persons having the benefit of this disclosure. Reference will now be made in detail to various implementations of the example embodiments as illustrated in the accompanying drawings. The same reference signs will be used to the extent possible throughout the drawings and the following description to refer to the same or like items.

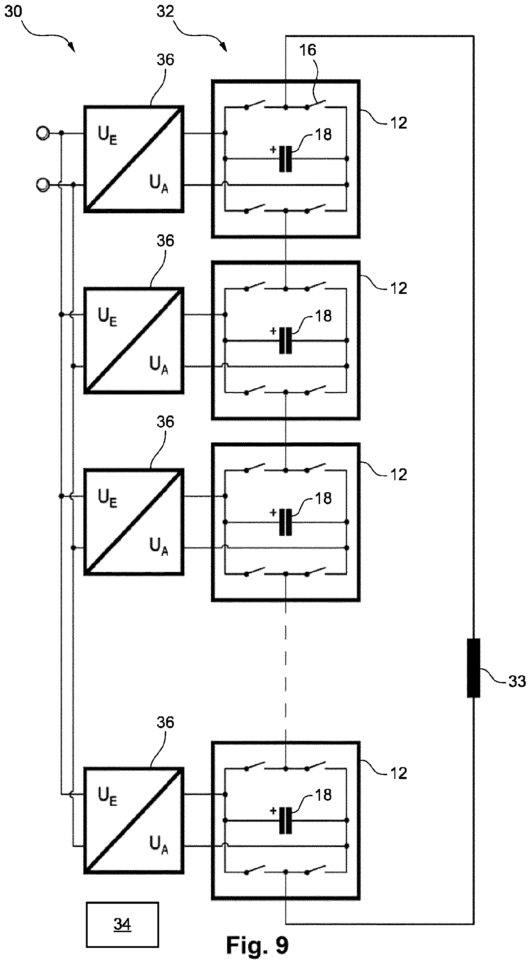

Fig. 9 shows a schematic view of a system 30 for generating a predetermined electromagnetic pulse. The system 30 comprises a voltage source 32 and a stimulation coil device 33. As is further shown in Fig. 9, the voltage source 32 comprises a chain of modules 12 and a control device 34. Each of the modules 12 comprises a capacitor 18, forming an example of the aforementioned energy storage device, and four switches 16, of which only an exemplary one is provided with reference a reference sign in Fig. 9. The interconnection of the capacitors 18 in the modules 12 collectively generates an output voltage of the voltage source 32 which is applied to said stimulation coil device 33. In other words, the "output voltage" is the voltage across the chain of modules 12 in the given switching states of the modules 12.

Under the control of the control device 34, the switches 16 can be operated such that each module 12 acquires a selected one of the following at least three operating states:

-     a series connection state, in which the capacitor 18 of the given module 12 is connected to introduce its module voltage to the voltage path along the chain of modules 12 with normal polarity to thereby positively contribute to the output voltage of the voltage source,

-     an anti-series connection state, in which the capacitor 18 of the given module 12 is connected to introduce its module voltage to the voltage path along the chain of modules 12 with reversed polarity to thereby negatively contribute to the output voltage of the voltage source, and

-     a bypass state, in which the module provides a conductive path therethrough without the capacitor 18 contributing to the output voltage of the voltage source.

These operating states can be chosen such that a desired output voltage of the voltage source 32 is obtained at any given point in time.

Moreover, each module 12 is connected with a charging voltage source 36, which in the embodiment shown is a controllable DC/DC converter. Under control of the control device 34, the charging voltage sources 36 allow for charging each of the capacitors 18 to acquire a corresponding "start voltage" which is determined in a manner described in more detail below.

The "control device" 34 as understood herein can be any control device suitable for controlling the switches 16 and the charging voltage sources 36. In particular, the control device 34 may comprise one or more microprocessors. In the alternative, the control device 34 could be a hardwired circuit, or could be an FPGA programmed to carry out the control functions described herein. In addition, the control device 34 may comprise a number of distributed and interconnected control units, for example dedicated module-control-units (not shown) which are each associated with a corresponding one of the modules 12, which module-control-units in turn are controlled by a main or central control unit (not shown). In some embodiments, the control device 34 can communicate with the switches 16 and the charging voltage sources 36 via signal lines (not shown for clarity in Fig. 9), while in other embodiments, the control device 34 could communicate with the switches 16/the charging voltage sources 36 by wireless communication.

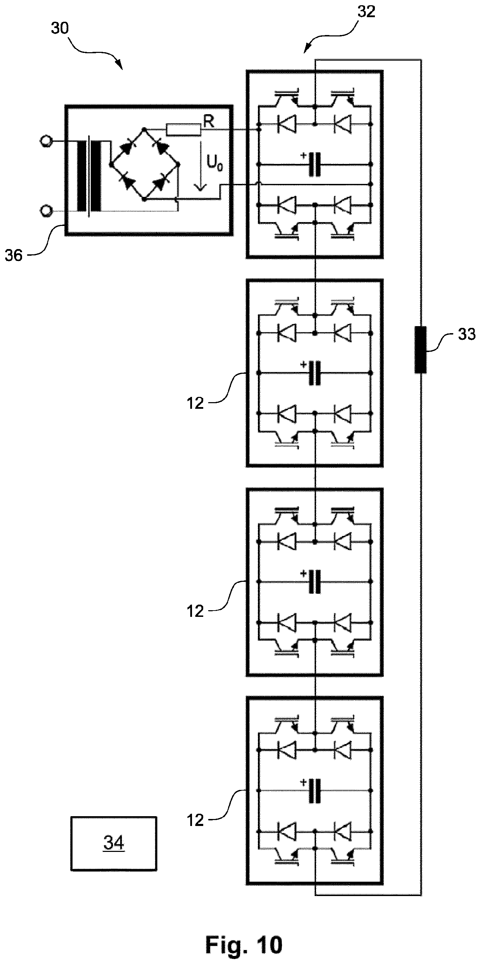

Fig. 10 shows a further embodiment of the system 30. In this embodiment, the switches 16, which are only represented by generic symbols in Fig. 9, are shown to be semiconductor switches with a reverse diode connected in parallel. In addition, an alternative variant of the charging voltage source 36 is shown.

The purpose of voltage source 32 is to provide a time-dependent output voltage to the coil device 33, to thereby generate a predetermined magnetic pulse, for example for TMS applications. Based on the impedance of the coil device 33, a suitable voltage profile can be determined which when applied to the coil device 33 generates the predetermined magnetic pulse. This suitable voltage profile is referred to herein as the "desired voltage pulse waveform".

In ordinary operation of an MMC or EMMC, this desired voltage pulse waveform would be resembled by at each instant in time connecting a suitable subset of capacitors 18 in series/anti-series, where the capacitors 18 would all be kept at a predetermined set voltage in operation. In the present embodiment, the control device 34 is configured to carry out a different control, in which the concept of fixed module voltages is abandoned. Instead, the control device 34 determines a set of "optimized" start voltages for each of the module capacitors, and during the pulse generation controls the switches such as to resemble the desired voltage pulse waveform as closely as possible, starting out from the optimized start voltages and without trying to keep the module voltages at their respective start voltages.

In a preferred, exemplary embodiment, the start voltages are determined in an iterative procedure described next:

For simplicity, it is assumed that there are only three modules, each having a corresponding module capacitor voltage U1, U2, and U3, respectively. In addition, the maximum value of the module voltages is assumed to be 10 V. The iterative procedure starts at a configuration where all the module voltages (voltages at capacitors 18) are set to the maximum value of 10 V.

Since each of the three modules can acquire a selected one out of three operating states, there are a total of $3^3 = 27$ switching states, that can be summarized in a matrix **S** as follows:

$$S = \begin{bmatrix} 1 & 1 & 1 \\ -1 & 1 & 1 \\ 0 & 1 & 1 \\ 1 & -1 & 1 \\ -1 & -1 & 1 \\ 0 & -1 & 1 \\ 1 & 0 & 1 \\ -1 & 0 & 1 \\ 0 & 0 & 1 \\ 1 & 1 & -1 \\ -1 & 1 & -1 \\ 0 & 1 & -1 \\ 1 & -1 & -1 \\ -1 & -1 & -1 \\ 0 & -1 & -1 \\ 1 & 0 & -1 \\ -1 & 0 & -1 \\ 0 & 0 & -1 \\ 1 & 1 & 0 \\ -1 & 1 & 0 \\ 0 & 1 & 0 \\ 1 & -1 & 0 \\ -1 & -1 & 0 \\ 0 & -1 & 0 \\ 1 & 0 & 0 \\ -1 & 0 & 0 \\ 0 & 0 & 0 \end{bmatrix}$$

In this matrix, the first, second and third columns denote the possible switching states of the first, second and third module respectively. The series connection state is represented by "1", the anti-series connection state is represented by "-1", and the bypass state is represented by "0".

When combining the voltages of the first, second and third modules 12 in a voltage vector $\vec{U}$,

$$\vec{U} = \begin{pmatrix} U1 \\ U1 \\ U3 \end{pmatrix}$$

a set of possible output voltages $\overrightarrow{U\,out}$ is obtained by multiplying the above matrix **S** with the voltage vector $\vec{U}$, i.e. $\overrightarrow{U\,out} = \mathbf{S}\,\vec{U}$.

When starting with $\vec{U} = \begin{pmatrix} U1 \\ U1 \\ U3 \end{pmatrix} = \begin{pmatrix} 10 \\ 10 \\ 10 \end{pmatrix}$ , the possible output voltages are hence

$$\mathbf{S} \begin{pmatrix} 10 \\ 10 \\ 10 \end{pmatrix} = \overrightarrow{U out} = \begin{pmatrix} 30 \\ 10 \\ 20 \\ 10 \\ -10 \\ 0 \\ 20 \\ 0 \\ 10 \\ 10 \\ -10 \\ 0 \\ -10 \\ -30 \\ -20 \\ 0 \\ -20 \\ -10 \\ 20 \\ 0 \\ 10 \\ 0 \\ -20 \\ -10 \\ 10 \\ -10 \\ 0 \end{pmatrix}$$

Using the maximum voltage of 10V for each of U1, U2 and U3 as the initial start voltages, the desired voltage pulse waveform can be resembled in a simulation. Herein, at each time step, a suitable one of the components of $\overrightarrow{U out}$ is chosen, typically the component deviating the least from the desired voltage pulse waveform at this point in time. The time steps could correspond to the switching frequency of the switches, and could correspond to 1 $\mu$s. The course of the output voltage is hence simulated, wherein in this simulation, a change of the voltage due to current flowing into or out of each of the capacitors 18 is taken into consideration. This means that the values of the components of $\overrightarrow{U out}$ will not be fixed at the start value, but will vary over time.

The result of the simulation is compared with the desired voltage pulse waveform. More precisely, an error voltage $V_E$ is determined which corresponds to the maximum deviation of the simulated time-dependent output voltage from the desired voltage pulse waveform. This "maximum deviation" corresponds to the maximum of the absolute values of the voltage differences between the time-dependent output voltage and the desired voltage waveform that occurs during the entire duration of the voltage pulse. With the "uneducated" start voltages, the deviations will be quite high, i.e. $V_E$ will be substantial. In order to reduce the deviation, improved start voltages are chosen by modifying start module voltages of the previous iteration step. In this embodiment, the start module voltages are iteratively modified by subtracting different voltage values from all of the start module voltages, which voltage values at least approximately add up to said error voltage $V_E$. In the specific embodiment, the start module voltage of the n-th module is reduced by $V_E/(2 \cdot n)$, for n = 1 ... N.

Let us assume that with the start voltages of 10V for each module, the error voltage turns out to be $V_E$ = 3V, and the average deviation of the simulated time-dependent output voltage from said desired voltage pulse waveform this 0.6V. The voltage values to be subtracted from the start voltages are hence determined for each module as follows:

Module#1:     3V/ (2*1) = 1.5 V, rounded to 1.5 V
Module#2:     3V/ (2*2) = 0.75 V, rounded to 0.8 V
Module#3:     3V/ (2*3) = 0.5 V, rounded to 0.5 V

The total corrected error hence is 2.8 V. In other words, the subtracted voltages approximately add up to the error voltage $V_E$ The modified start voltages are then obtained as follows:

$$\vec{U} = \begin{pmatrix} 10 \\ 10 \\ 10 \end{pmatrix} - \begin{pmatrix} 1,5 \\ 0,8 \\ 0,5 \end{pmatrix} = \begin{pmatrix} 8,5 \\ 9,2 \\ 9,5 \end{pmatrix}$$

With these modified voltages, the possible output voltages are again found by multiplying the voltage vector with the matrix **S**, i.e.

$$\mathbf{S}\begin{pmatrix} 8.5 \\ 9.2 \\ 9.5 \end{pmatrix} = \overrightarrow{Uout} = \begin{pmatrix} 27,2 \\ 10,2 \\ 18,7 \\ 8,8 \\ -8,2 \\ 0,3 \\ 18 \\ 1 \\ 9,5 \\ 8,2 \\ -8,8 \\ -0,3 \\ -10,2 \\ -27,2 \\ -18,7 \\ -1 \\ -18 \\ -9,5 \\ 17,7 \\ 0,7 \\ 9,2 \\ -0,7 \\ -17,7 \\ -9,2 \\ 8,5 \\ -8,5 \\ 0 \end{pmatrix}$$

With the new start voltages, in the next step of the iteration, it is again tried to resemble the desired voltage pulse waveform. Assume that in this second round, the value of the error voltage $V_E$ is found to be 1.2 V, and the average deviation is 0.1 V. The voltage values to be subtracted from the start voltages are hence determined for each module as follows:

|           |                                      |
|-----------|--------------------------------------|
| Module#1: | 1.2V/ (2*1) = 0.6 V, rounded to 0.6 V |
| Module#2: | 1.2V/ (2*2) = 0.3 V, rounded to 0.3 V |
| Module#3: | 1.V/ (2*3) = 0.2 V, rounded to 0.2 V  |

The total corrected error is hence (0.6+0.3+0.2) = 1.1V. In other words, the subtracted voltages approximately add up to the error voltage $V_E$.

This procedure is repeated until some stop criterion is met. For example, the procedure may be terminated once the average deviation of the simulated time-dependent output voltage from said desired voltage pulse waveform exceeds a threshold value. In the alternative, the procedure may be stopped if the average deviation stops decreasing for a given number of successive iterations.

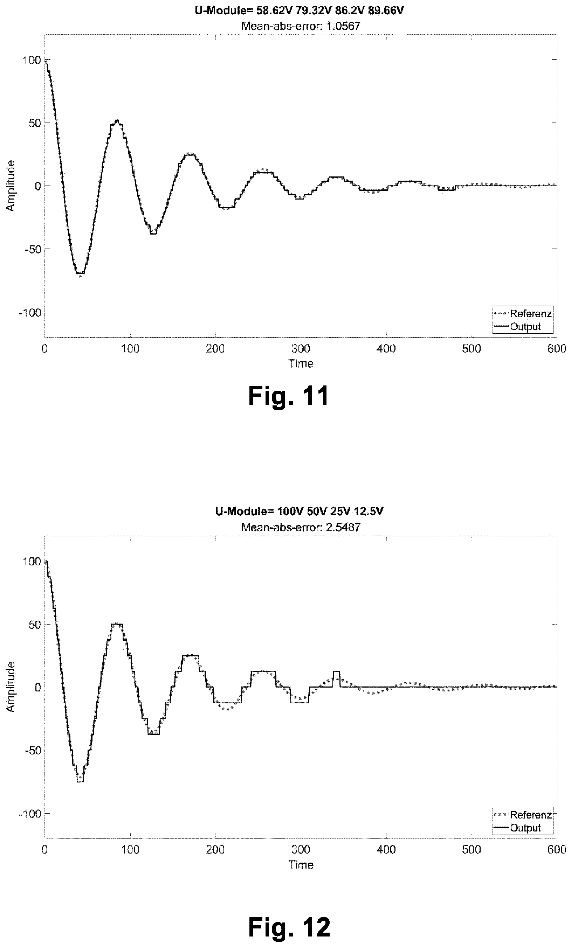

It is found that with this procedure, it is possible to reduce the deviations from the desired voltage pulse waveform significantly as compared to the case where MMCs or EMMCs under conventional control are used. This is demonstrated with reference to Fig. 11 to 18, as will be described next.

Fig. 11 shows a damped cosine as an exemplary desired voltage pulse waveform, serving as a reference and represented by a dotted line. The amplitude of the undamped cosine is 100 V, which corresponds to the aforementioned voltage $V_{pulse,max}$, i.e. the maximum of the absolute value of the desired voltage pulse waveform. Note that in the present disclosure, the term "pulse" has a broad meaning which also encompasses signals having several peaks and/or being of oscillatory nature, as shown in Fig. 11, with six positive and six negative peaks. Indeed, pulses having damped oscillatory shapes are common in existing devices. However, with the method of the invention, it is possible to generate practically arbitrary photos pulse waveforms, including those shown e.g. in Fig. 4. The unit on the horizontal axis is "time steps", which corresponds to the inverse switching frequency, and could e.g.be on the order of 1 microsecond. Further shown in solid line is a time-dependent output voltage resembling the same, which was generated with a method according to an embodiment of the invention using a voltage source having four modules.

When generating the time-dependent output voltage, the start voltages have been determined in the iterative optimization procedure described above. The thus determined start voltages amount to 58.62 V, 59.32 V, 86.2 V, and 89.66 V. With these start voltages, it is possible to very closely match the desired voltage pulse waveform with the time-dependent output voltage, with a mean absolute error of 1.06 V only. Herein, the "mean deviation" or "mean absolute error" is the arithmetic mean of the absolute values of the voltage differences between the time-dependent output voltage and the desired voltage waveform determined at a number of discrete time steps, such as the time steps at which the module switching is carried out. This is an example of the time steps "corresponding to the switching frequency of the switching devices" referred to above, with the time step duration being essentially the inverse of the switching frequency.

For comparison, Fig. 12 shows the time-dependent output voltage obtained when trying to reproduce the same damped-cosine desired voltage pulse waveform with a conventional EMMC having four modules as well. As was explained above, such four-module EMMC can generate $2^4+1 = 17$ evenly spaced voltage levels. In this case, the voltage of the highest voltage module has been chosen to correspond to $V_{pulse,max} = 100$ V (with the further module voltages being 50 V, 25 V and 12.5 V), so that most of the 17 voltage levels can actually be employed.

If $V_{pulse,max}$ of the desired voltage pulse waveform were for example only 50 V, then at most half of the available voltage steps could have been be used. In other words, in this comparative example, the voltages of the EMMC modules are optimally adapted to the desired voltage pulse waveform, a luxury that will typically not exist when employing the EMMC for the generation of various or arbitrary pulses. Still, it is seen that owing to the limited number of voltage levels, the time-dependent output voltage deviates more from the desired voltage pulse waveform than in case of the method according to an embodiment of the invention as shown in Fig. 11. This is seen with the bare eye, but also apparent from the mean absolute error, which in this case is 2.55 V. In other words, using the method according to an embodiment of the invention, for the same number of modules, the "average deviation" can be reduced to 1.06:2.55 = 42%.

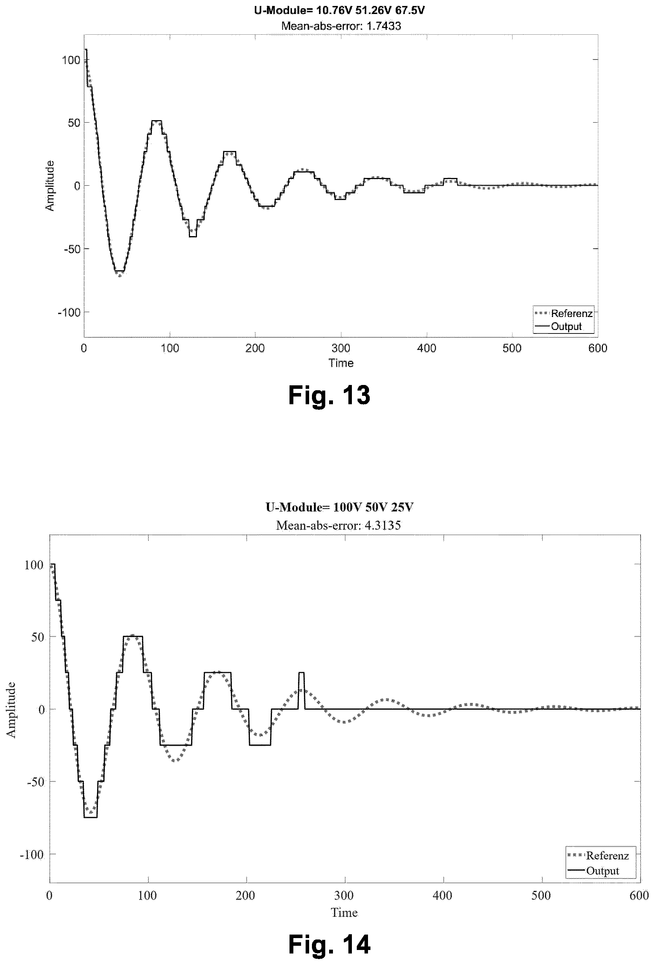

Fig. 13 and Fig. 14 show the same desired voltage pulse waveform as Fig. 11 and Fig. 12 (broken line). Moreover, Fig. 13 shows a corresponding time-dependent output voltage (solid line) generated with the method according to an embodiment of the invention using a voltage source having three modules. In the aforementioned optimization procedure, start voltages of 10.76 V, 51.26 V, and 67.5 V were determined. Fig. 14 shows a corresponding time-dependent output voltage generated with a conventional EMMC having three modules as well, with module voltages of 100 V, 50 V, and 25 V. Again it is seen that with the method according to an embodiment of the invention, the "average deviation" between the generated time-dependent output voltage and the desired voltage pulse waveform is reduced from 4.31 V for the EMMC of Fig. 14 to 1.74 in case the embodiment of the invention (Fig. 13), which corresponds to a reduction of 1.74:4.31 = 40%.

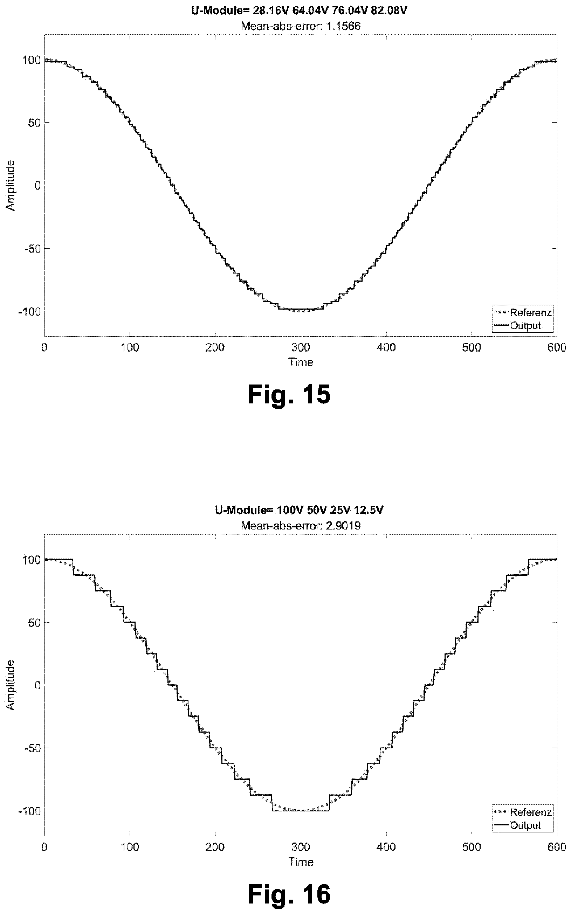

Fig. 15 to 18 are similar to Fig. 11 to 14, except that in this case a cosine is used as an exemplary desired voltage pulse waveform.

Fig. 15 and 16 show the corresponding time-dependent output voltage, generated with the method according to an embodiment of the invention (Fig. 15) and with an EMMC under its normal operation (Fig. 16), where in each case four modules were employed. As before, the start voltages are obtained via the aforementioned optimization procedure. The determined start voltages, the fixed voltages of the EMMC and the corresponding mean absolute errors are recited in the figures. It is seen that the optimized start voltages in case of Fig. 15 (28.16 V, 64.04 V, 76.04 V, and 82.08 V) are very different from those of Fig. 11, which demonstrates that indeed, the start voltages have to be adapted to each given desired voltage pulse waveform. It is seen that the "average deviation" between the generated time-dependent output voltage and the desired voltage pulse waveform is reduced from 2.90 V of the EMMC of Fig. 16 to 1.16 in case an embodiment of the invention (Fig. 15), corresponding to a reduction of 1.16 : 2.90 = 40%.

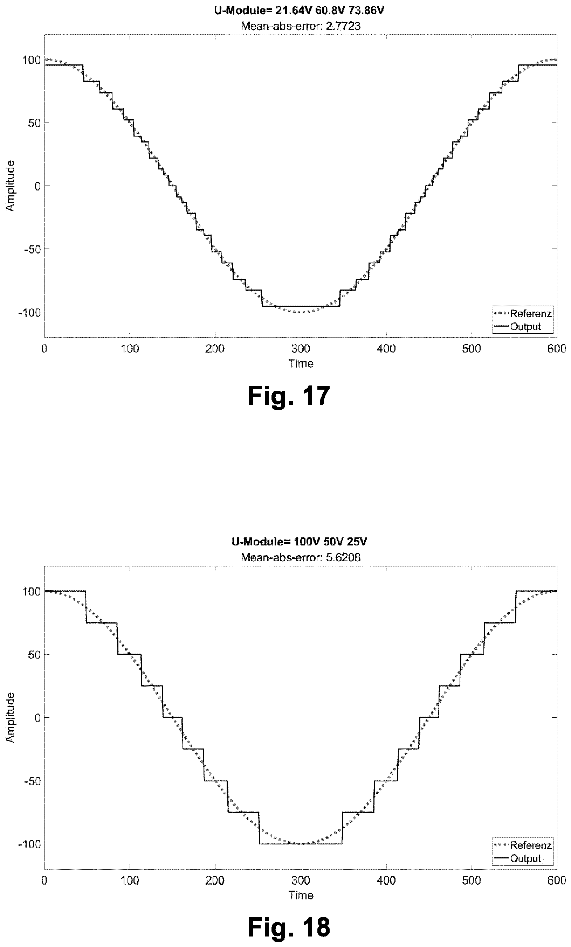

Fig. 17 and 18 are similar to Fig. 15 and 16, except that the voltage source/EMMC in this case has three modules only. As compared to the mean absolute error ("average deviation") of 5.62 V obtained with the EMMC of Fig. 18, the mean absolute error in case of the embodiment of the invention (Fig. 17) is reduced to 2.77 V, which amounts to a reduction of 2.77:5.62 = 49%.

It is therefore seen that for the same number of modules, the method of the invention allows for smoother voltage curves and a better matching between the generated time-dependent output voltage and the desired voltage pulse waveform as in case of the EMMC. Indeed, the mean absolute error in case of only three modules using the invention is still less than in case of an EMMC having four modules. This better performance is due to the fact that depending on the choice of start voltages, the system and method of the invention in principle allow $3^N$ different voltage levels. Moreover, the thus obtainable voltage levels do not need to be evenly spaced, instead, a dense spacing of the obtainable voltage levels can be reserved for portions of the desired voltage pulse waveform, where it is truly needed, which is typically in regions where the slope of the desired voltage pulse waveform is small.

While the present invention has been described in terms of specific embodiments, it is understood that variations and modifications will occur to those in the art, all of which are intended as aspects of the present invention. Accordingly, only such limitations as appear in the claims should be placed on the invention.

LIST OF REFERENCE SIGNS

| | |
|---|---|
| 10 | leading edge of pulse |
| 12 | module |
| 14 | terminal |
| 16 | switching device |
| 18 | capacitor |
| 22 | converter arm |
| 24 | first end of converter arm 22 |
| 26 | second end of converter arm 22 |
| 30 | system for generating a predetermined electromagnetic pulse |
| 32 | voltage source |
| 33 | stimulation coil device |
| 34 | control device |
| 36 | charging voltage source |

**Claims**

1. A method of generating a predetermined electromagnetic pulse using a voltage source (32) and a load (33) connected thereto,

   wherein the voltage source (32) comprises a chain of modules (12), in which a number of N modules are connected in series, with $N \geq 3$, and wherein a voltage across said chain of modules (12) represents an output voltage of the voltage source (32) to be applied to said load (33),
   wherein each of said modules (12) comprises an energy storage device (18) having a module voltage and a plurality of switching devices (16), wherein said switching devices (16) are configured to allow for selectively establishing each of the following at least three operating states:

   - a series connection state, in which the energy storage device (18) is connected to introduce its module voltage to the voltage path along the chain of modules (12) with normal polarity to thereby positively contribute to the output voltage of the voltage source (32),
   - an anti-series connection state, in which the energy storage device (18) is connected to introduce its module voltage to the voltage path along the chain of modules (12) with reversed polarity to thereby negatively contribute to the output voltage of the voltage source (32), and
   - a bypass state, in which the module (12) provides a conductive path therethrough without the energy storage device (18) contributing to the output voltage of the voltage source (32),

   the method comprising the steps of:

   - determining a desired voltage pulse waveform which is suitable for generating said predetermined electromagnetic pulse when applied to said load (33),
   - determining an individual start module voltage for each of said N modules, based on said desired voltage pulse waveform,
   - charging the energy storage device (18) of each module (12) such as to acquire the corresponding determined start module voltage, and
   - generating a time-dependent output voltage at the voltage source resembling said desired voltage pulse waveform by selectively switching the switching devices (16) of each of the modules (12) to acquire a selected one of said at least three operating states as a function of time, and applying said time-dependent output voltage to said load (33),

wherein said step of determining said individual start module voltage for each of said N modules comprises an optimization procedure, in which said start module voltages are determined such as to reduce a deviation between the generated time-dependent output voltage and the desired voltage pulse waveform.

2. The method of claim 1, wherein said predetermined electromagnetic pulse is a magnetic stimulation pulse and said load (33) is a stimulation coil device (33).

3. The method of claim 1 or 2, wherein the optimization procedure is such that with the thus determined start module voltages, one or both of the maximum and average deviation between the time-dependent output voltage and the desired voltage pulse waveform is less than the maximum and average deviation, respectively, obtainable with a voltage source (32) having $2^N +1$ evenly distributed voltage states between - $V_{pulse,max}$ and + $V_{pulse,max}$, preferably less than 75%, more preferably less than 50% of said maximum or average deviation, respectively, wherein $V_{pulse,max}$ is the maximum of the absolute value of the desired voltage pulse waveform.

4. The method of one of the preceding claims, wherein during said step of generating said time-dependent output voltage, the energy storage devices (18) are permitted to change their voltage due to energy transferred to the load (33) and between modules, and this change of voltage is accounted for in the step of determining said set of individual start module voltages.

5. The method of one of the preceding claims, wherein said optimization procedure comprises the steps of

   a) simulating the time-dependent output voltage that would be obtained with a given set of start module voltages,
   b) determining a deviation indicator indicating a deviation of the simulated time-dependent output voltage from the desired voltage pulse waveform, and
   c) modifying some or all of the start module voltages based on said determined deviation indicator,

   wherein the simulation in said step a) preferably comprises estimating the time-dependent current flow into the load (33) and accounting for the change in module voltages associated therewith, and/or wherein said steps a) to c) are preferably conducted iteratively until a stop criterion is met, wherein preferably, said stop criterion is a convergence criterion, and in particular, a criterion in which the average deviation of the simulated time-dependent output voltage from said desired voltage pulse waveform exceeds a threshold value or stops decreasing for a given number of successive iterations.

6. The method of claim 5, wherein in step b), an error voltage $V_E$ is determined, which corresponds to the maximum deviation of the simulated time-dependent output voltage from said desired voltage pulse waveform, said maximum deviation corresponding to the maximum of the absolute values of the voltage differences between the time-dependent output voltage and the desired voltage waveform that occurs during the entire duration of the voltage pulse, and in step c), the start module voltages are modified by subtracting different voltage values from all or at least some of the start module voltages which at least approximately add up to said error voltage $V_E$, wherein in step c), the start module voltage of the $n$-th module is preferably reduced by $V_E./(2 \cdot n)$ , for $n = 1 ... N$.

7. The method of one of the preceding claims, wherein $10 \geq N \geq 3$, preferably $8 \geq N \geq 4$, and/or wherein said energy storage device (18) is a capacitor (18), and/or wherein said switching devices (16) comprise transistor switching devices (16), in particular MOSFET or IGBT switching devices (16).

8. A system (30) for generating a predetermined electromagnetic pulse, the system (30) comprising a voltage source (32) and a load (33) connected thereto,

   wherein the voltage source (32) comprises
   a control device (34), and
   a chain of modules (12), in which a number of N modules are connected in series, with $N \geq 3$, and wherein a voltage across said chain of modules (12) represents an output voltage of the voltage source (32) to be applied to said load (33),
   wherein each of said modules (12) comprises an energy storage device (18) having a module voltage and a plurality of switching devices (16), wherein said switching devices (16) are configured to allow for selectively establishing, under control of said control device (34), each of the following at least three operating states:

      - a series connection state, in which the energy storage device (18) is connected to introduce its module voltage to the voltage path along the chain of modules (12) with normal polarity to thereby positively contribute to the output voltage of the voltage source (32),

- an anti-series connection state, in which the energy storage device (18) is connected to introduce its module voltage to the voltage path along the chain of modules (12) with reversed polarity to thereby negatively contribute to the output voltage of the voltage source (32), and

- a bypass state, in which the module (12) provides a conductive path therethrough without the energy storage device (18) contributing to the output voltage of the voltage source (32),

wherein said control device (34) is further configured for receiving information representing a desired voltage pulse waveform which is suitable for generating said predetermined electromagnetic pulse when applied to said load (33), or for determining such desired voltage pulse waveform, wherein said control device (34) is further configured for

- determining an individual start module voltage for each of said N modules, based on said desired voltage pulse waveform,

- controlling a charging of the energy storage device (18) of each module (12) such as to acquire the corresponding determined start module voltage, and

- controlling the switching elements such as to selectively switch the switching devices (16) of each of the modules (12) to acquire a selected one of said at least three operating states as a function of time, to thereby generate a time-dependent output voltage at the voltage source resembling said desired voltage pulse waveform,

wherein said control device (34) is configured for determining said individual start module voltage for each of said N modules using an optimization procedure, in which said start module voltages are determined such as to reduce a deviation between the generated time-dependent output voltage and the desired voltage pulse waveform.

9. The system (30) of claim 8, wherein said predetermined electromagnetic pulse is a magnetic stimulation pulse and said load (33) is a stimulation coil device (33).

10. The system (30) of claim 8 or 9, wherein the optimization procedure is such that with the thus determined start module voltages, one or both of the maximum and average deviation between the time-dependent output voltage and the desired voltage pulse waveform is less than the maximum and average deviation, respectively, obtainable with a voltage source (32) having $2^N +1$ evenly distributed voltage states between $- V_{pulse,max}$ and $+ V_{pulse,max}$, preferably less than 75%, more preferably less than 50% of said maximum or average deviation, respectively, wherein $V_{pulse,max}$ is the maximum of the absolute value of the desired voltage pulse waveform..

11. The system (30) of one of claims 8 to 10, wherein said control device (34) is configured to control said switches such that during said step of generating said time-dependent output voltage, the energy storage devices (18) are permitted to change their voltage due to energy transferred to the load (33) and between modules, wherein said control device (34) is configured to account for this change of voltage in the step of determining said set of individual start module voltages.

12. The system (30) of one of claims 8 to 11, wherein said optimization procedure comprises the steps of

a) simulating the time-dependent output voltage that would be obtained with a given set of start module voltages,
b) determining a deviation indicator indicating a deviation of the simulated time-dependent output voltage from the desired voltage pulse waveform, and
c) modifying some or all of the start module voltages based on said determined deviation indicator,

wherein the simulation in said step a) preferably comprises estimating the time-dependent current flow into the load (33) and accounting for the change in module voltages associated therewith.

13. The system (30) of claim 12, wherein the control device (34) is configured to conduct said steps a) to c) iteratively until a stop criterion is met, wherein preferably, said stop criterion is a convergence criterion, and in particular, a criterion in which the average deviation of the simulated time-dependent output voltage from said desired voltage pulse waveform exceeds a threshold value or stops decreasing for a given number of successive iterations.

14. The system (30) of claim 12 or 13, wherein said control device (34) is configured for determining, in step b), an error voltage $V_E$, which corresponds to the maximum deviation of the simulated time-dependent output voltage from said desired voltage pulse waveform, said maximum deviation corresponding to the maximum of the absolute values of the voltage differences between the time-dependent output voltage and the desired voltage waveform that occurs during the entire duration of the voltage pulse, and to modify, in step c), the start module voltages by subtracting different voltage values from all or at least some of the start module voltages which at least approximately add up to said error voltage $V_E$, wherein said control device (34) is preferably configured to reduce, in step c), the start module voltage of the $n$-th module by $V_E./(2 \cdot n)$, for n = 1 ... N.

15. The system (30) of one of claims 8 to 14, wherein $10 \geq N \geq 3$, preferably $8 \geq N \geq 4$, and/or wherein said energy storage device (18) is a capacitor (18), and/or wherein said switching devices (16) comprise transistor switching devices (16), in particular MOSFET or IGBT switching devices (16).

**Fig. 1**

**Fig. 2**

**Fig. 3**

**Fig. 4**

**Fig. 5**

| | Outputvoltage Ua in V | | | | | | | | | | | | | | | |
|---|---|---|---|---|---|---|---|---|---|---|---|---|---|---|---|---|
| | 0 | 50 | 100 | 150 | 200 | 250 | 300 | 350 | 400 | 450 | 500 | 550 | 600 | 650 | 700 | 750 |
| 400 V | | | | | | | | | x | x | x | x | x | x | x | x |
| 200 V | | | | | x | x | x | x | | | | | x | x | x | x |
| 100 V | | | x | x | | | x | x | | | x | x | | | x | x |
| 50 V | | x | | x | | x | | x | | x | | x | | x | | x |

x: Modul on

## Fig. 6

EP 4 614 813 A1

Fig. 7

Fig. 8

**Fig. 9**

**Fig. 10**

**U-Module= 58.62V 79.32V 86.2V 89.66V**
Mean-abs-error: 1.0567

## Fig. 11

**U-Module= 100V 50V 25V 12.5V**
Mean-abs-error: 2.5487

## Fig. 12

**U-Module= 10.76V 51.26V 67.5V**
Mean-abs-error: 1.7433

# Fig. 13

**U-Module= 100V 50V 25V**
Mean-abs-error: 4.3135

# Fig. 14

**U-Module= 28.16V 64.04V 76.04V 82.08V**
Mean-abs-error: 1.1566

**Fig. 15**

**U-Module= 100V 50V 25V 12.5V**
Mean-abs-error: 2.9019

**Fig. 16**

**U-Module= 21.64V 60.8V 73.86V**
Mean-abs-error: 2.7723

**Fig. 17**

**U-Module= 100V 50V 25V**
Mean-abs-error: 5.6208

**Fig. 18**

Europäisches
Patentamt

European
Patent Office

Office européen
des brevets

# EUROPEAN SEARCH REPORT

**Application Number**

EP 24 16 1850

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/030622 A1 (WEYH THOMAS [DE] ET AL) 30 January 2020 (2020-01-30) | 1-5, 7-13,15 | INV. H03K3/57 |
| A | * paragraphs [0066] - [0073]; figure 9 * <br> * paragraphs [0074] - [0077]; figure 10a * <br> * paragraphs [0078], [0079]; figure 10b * | 6,14 | A61N2/02 |
| X | LI Z ET AL: "Modular pulse synthesizer for transcranial magnetic stimulation with fully adjustable pulse shape and sequence", JOURNAL OF NEURAL ENGINEERING, IOP PUBLISHING, BRISTOL, GB, vol. 19, no. 6, 23 November 2022 (2022-11-23), XP020469107, ISSN: 1741-2560, DOI: 10.1088/1741-2552/AC9D65 [retrieved on 2022-11-23] | 1-5, 7-13,15 | |
| A | * Sections 2.4 and 2.5; figure 1(D) * | 6,14 | |

**TECHNICAL FIELDS SEARCHED (IPC)**

H03K
A61N

The present search report has been drawn up for all claims

| Place of search | Date of completion of the search | Examiner |
|---|---|---|
| Munich | 5 August 2024 | Martínez Martínez, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 16 1850

This annex lists the patent family members relating to the patent documents cited in the above-mentioned European search report.
The members are as contained in the European Patent Office EDP file on
The European Patent Office is in no way liable for these particulars which are merely given for the purpose of information.

05-08-2024

| Patent document cited in search report | Publication date | Patent family member(s) | Publication date |
|---|---|---|---|
| US 2020030622 A1 | 30-01-2020 | DE 102017108084 A1 | 18-10-2018 |
| | | DE 202017102723 U1 | 01-06-2017 |
| | | US 2020030622 A1 | 30-01-2020 |
| | | WO 2018189387 A1 | 18-10-2018 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 7753836 B2
- US 7946973 B1
- DE 102012101921 A1

- DE 10217889 A1
- DE 102017108099 A1
- DE 102017108084 A1

### Non-patent literature cited in the description

- **A. V. PETERCHEV et al.** Repetitive Transcranial Magnetic Stimulator with Controllable Pulse Parameters. *Journal of Neural Engineering*, 2011, vol. 8 (3), 036016
- **A. V. PETERCHEV**. Circuit Topology Comparison and Design Analysis for Controllable Pulse Parameter Transcranial Magnetic Stimulators. *Proceedings of the 5th International IEEE EMBS Conference on Neural Engineering*, 2011, 646
- **S. M. GÖTZ et al.** Analysis and Optimisation of Pulse Dynamics for Magnetic Stimulation. *arxiv: 1106.3452v1*, 17 June 2011

- **S. ALLEBROD** ; **R. HAMERSKI** ; **R. MARQUARDT**. New transformerless, scalable modular multilevel converters for hvdc-transmission,. *2008 IEEE Power Electronics Specialists Conference*, June 2008, 174-179
- **KUDER, M.** ; **KERSTEN, A.** ; **BERGMANN, L. et al.** Exponential Modular Multilevel Converter for Low Voltage Applications. *2019 21st European Conference on Power Electronics and Applications, EPE 2019 ECCE Europe*, 2019, 1-11, http://dx.doi.org/10.23919/EPE.2019.8915156